# FASCICULE DE BREVET EUROPEEN

(11) **EP 1 508 316 B1**
(45) Date de publication et mention de la délivrance du brevet: **16.05.2007**
(21) Numéro de dépôt: 04290319.5
(22) Date de dépôt: 06.02.2004
(51) Int. Cl.: A61F 2/42

(54) **Prothèse totale métatarso-phalangienne, et ancillaires pour la mise en place de cette prothèse**
Metatarsalphalangeale Vollprothese und Hilfsgerät zum Einsetzen einer solchen Prothese.
Total metatarsalphalangeal prothesis and ancillary for setting same.

(30) Priorité: 06.08.2003 FR 0309707
(43) Date de publication de la demande: 23.02.2005
(73) Titulaire: Depuy (Ireland) Limited, Ringaskiddy, County Cork (IE); Pied Innovation 2 "P.1.2", 69160 Tassin la Demi Lune (FR)
(72) Inventeur: Augoyard, Marc, 69160 Tassin la Demi-Lune (FR); Benichou, Michel, 34070 Montpellier (FR); Leemrijse, Thibaut, 1200 Bruxelles (BE); Maestro, Michel, 06200 Nice (FR); Peyrot, Jacques, 69160 Tassin la Demi-Lune (FR); Ragusa, Mathieu, 38240 Grenoble (FR); Valtin, Bernard, 94500 Champigny (FR); Poncet, Didier, 69500 Bron (FR)
(74) Mandataire: Bernasconi, Jean Raymond

(56) Documents cités:
- WO-A-01/03613
- WO-A-02/43627
- DE-A- 10 130 796
- FR-A- 2 787 013
- FR-A- 2 803 509
- US-A- 5 458 648
- US-A- 5 601 565
- US-A- 5 843 085
- US-A- 6 030 391

## Description

La présente invention a trait à une nouvelle prothèse totale métatarso-phalangienne, dite à coupe, notamment pour l'articulation entre le premier métatarsien et la première phalange du gros orteil, ainsi qu'à des ancillaires pour la mise en place de cette prothèse.

Elle a également pour objets un jeu d'implants métatarsiens et un jeu de telles prothèses de dimensions différentes permettant au chirurgien un choix per-opératoire des dimensions des différents constituants de la prothèse.

On connaît déjà des prothèses totales métatarso-phalangiennes comprenant un implant métatarsien présentant une tige d'ancrage osseuse et un corps articulaire disposé à l'extrémité de cette tige, un implant phalangien et un plot ou plateau intermédiaire destiné à être disposé sur l'implant phalangien et présentant une surface articulaire complémentaire de la surface articulaire de l'implant métatarsien.

Une telle prothèse est, par exemple, décrite dans le brevet FR-B-2 787 013.

La présente invention se propose, d'une façon générale, d'améliorer les prothèses totales métatarso-phalangiennes. Elle se propose en particulier de simplifier ces prothèses et de leur conférer de meilleures propriétés anatomiques améliorant la fonctionnalité de l'articulation métatarso-phalangienne établie par une prothèse ainsi que l'adaptation de cette prothèse dans le milieu anatomique de cette articulation.

L'invention se propose également de fournir des ancillaires permettant la mise en place précise des implants, dans tous les cas de figure rencontrés.

L'invention a pour objet une prothèse totale métatarso-phalangienne comprenant

un implant métatarsien comprenant une tige d'ancrage destinée à être disposée longitudinalement dans le métatarsien, et une tête située à l'extrémité de la tige, destiné à être appliquée contre la surface de la coupe de l'extrémité du métatarsien, et présentant une surface articulaire,
et un implant phalangien présentant un corps destiné à être introduit axialement dans la phalange et une margelle située à l'extrémité proximale de ladite partie et susceptible de présenter une surface articulaire complémentaire de la surface articulaire présentée par le corps de l'implant métatarsien,
ladite surface articulaire de l'implant phalangien pouvant, de préférence, être portée par un plot distinct susceptible d'être introduit et maintenu dans l'implant phalangien,
caractérisé en ce que la tête de l'implant métatarsien présente, à l'opposé de la surface articulaire, une face plane arrière inclinée, destinée à venir s'appliquer contre une surface de coupe du métatarsien, ladite face arrière formant, avec le plan perpendiculaire à l'axe de la tige d'ancrage, un angle de 20° ± 5° pour s'étendre en arrière vers le haut, depuis la base inférieure du métatarsien,
et en ce que la surface articulaire de ladite tête de ladite face arrière inclinée se rencontrent en un point inférieur, formant le point le plus bas de l'implant métatarsien, la distance entre l'axe de la tige d'ancrage et ledit point le plus bas étant déterminée pour que la tête d'implant ne s'étende pas dans les gorges sésamoïdes disposées sur la base inférieure du métatarsien.

Il en résulte que lorsque le pied est posé à plat sur le sol, la face arrière de la tête de l'implant métatarsien se trouve dans une position sensiblement perpendiculaire au sol. Par ailleurs, la liberté laissée au niveau des gorges sésamoïdes permet de conserver la fonctionnalité des ligaments et de l'articulation.

Dans une forme de réalisation préférée, la surface articulaire du corps de l'implant ne présente qu'un seul rayon dans le plan sagittal. En d'autres termes, une section sagittale de cette surface forme un arc de cercle. Egalement de façon très avantageuse, ladite surface peut aussi ne présenter, dans le plan horizontal, qu'un seul rayon.

Bien entendu la surface articulaire en regard de l'implant phalangien est complémentaire.

De façon particulièrement préférée, la surface articulaire complémentaire est formée par un plot intermédiaire, porté par l'implant phalangien, et libre en rotation autour de l'axe de la phalange.

On obtient ainsi une parfaite congruence dans toutes les positions, le plot rotatif s'adaptant en permanence dans toutes les positions angulaires.

La surface articulaire du plot peut avantageusement présenter, sur sa périphérie, et plus précisément sur une partie sensible de la périphérie de la moitié inférieure et/ou de la périphérie de la moitié supérieure, une échancrure déterminant un rebord convexe pour le dégagement de l'articulation en position de flexion et/ou d'extension.

Dans une forme de réalisation préférée de l'invention, et notamment dans le cas où la surface articulaire phalangienne est formée par un plot tournant librement dans l'implant phalangien, ce dernier présente un corps d'ancrage tronconique creux se poursuivant, à son extrémité proximale, par une tête en forme de margelle tronconique périphérique, le plot présentant une forme correspondante, à savoir une partie distale tronconique dont la conicité correspond à celle du corps, se poursuivant par un plateau ayant une surface distale périphérique dont la conicité correspond à celle de la margelle de l'implant, ce qui permet également de donner au plot une forme particulièrement résistante.

A la périphérie, la margelle peut venir au contact de la corticale de la phalange, et y prendre appui.

De préférence, la face proximale inclinée à 20° ± 5° de la tête de l'implant métatarsien se prolonge, vers le haut, par une face supérieure formant, avec la précédente, un dièdre, de préférence d'un angle obtus de 145° ± 20°, destiné à être appliqué contre un plan de coupe supérieur incliné du même angle par rapport à la coupe recevant la face proximale.

L'invention a aussi pour objet un jeu d'implants métatarsiens, comprenant plusieurs implants métatarsiens de tailles différentes, adaptés pour former une partie de la prothèse définie ci-dessus, la distance entre l'axe de la tige d'ancrage et le point le plus bas étant sensiblement constante pour tous les implants du jeu.

Les gorges sésanoïdiennes restent ainsi dégagées, quelque soit l'implant métatarsien utilisé.

Dans ce jeu, les faces articulaires des têtes des implants métatarsiens sont de préférence dimensionnées pour rencontrer la face supérieure correspondante en un point supérieur à des distances respectives prédéterminées différentes de l'axe de la tige d'ancrage.

Lors de pose de la prothèse, il est aussi possible de choisir un implant métatarsien dont la partie supérieure est adaptée au mieux à l'état ligamentaire du niveau supérieur de l'articulation. Ces ligaments sont en effet souvent déformés, figés ou durcis par la pathologie qui a nécessité la pose de la prothèse. L'implant métatarsien sera notamment choisi pour ne pas gêner les ligaments latéraux lors de la flexion dorsale de l'articulation.

Avantageusement, le diamètre de la tige de l'implant métatarsien peut être sensiblement constant, quel que soit l'implant du jeu et l'on peut, éventuellement, faire simplement varier la longueur de cette tige d'un implant à l'autre dans le jeu.

L'invention a également pour objet un jeu de prothèses métatarso-phalangiennes présentant tout ou partie des caractéristiques de la prothèse définie ci-dessus.

Un jeu de prothèses selon l'invention, et qui comprend plusieurs implants métatarsiens d'un jeu tel que défini ci-dessus, plusieurs implants phalangiens de tailles différentes et plusieurs plots, de tailles également différentes, se caractérise par le fait que le plot dont la surface articulaire est congruente avec la surface articulaire d'un implant métatarsien donné, peut être reçu dans, au moins, deux implants phalangiens de tailles consécutives.

Ceci permet en outre de procéder à la même découpe en dièdre en utilisant les mêmes ancillaires, quelle que soit la taille du métatarsien.

L'implant phalangien peut présenter un corps tronconique ayant la même dimension pour tous les implants dans le jeu, ce cône étant suivi de la margelle tronconique, seul le diamètre de la périphérie de cette margelle étant variable d'un implant à l'autre dans le jeu de façon, de préférence, à venir s'appliquer contre la corticale phalangienne en étant cependant en retrait par rapport à l'encombrement extérieur de la phalange au niveau de la coupe.

On peut définir plusieurs plots dans le jeu, ayant tous la même partie tronconique de plot adaptée au corps tronconique de l'implant phalangien et ayant des diamètres périphériques et/ou des épaisseurs de plateau différents dans le jeu.

Le jeu d'implants est de préférence présenté en kits stériles.

Dans une forme de réalisation particulière, du jeu selon l'invention, on prévoit trois ou quatre tailles d'implants métatarsiens ayant des tiges de 5mm de diamètre. Par exemple pour un jeu comprenant trois implants métatarsiens, les rayons de courbure de la surface articulaire 10 sont respectivement de 7 ± 0,5, 8 ± 0,5 et 9 ± 0,5 mm dans le plan sagittal et 14 ± 1, 16 ± 1 et 18 ± 1 mm dans le plan horizontal, et les points les plus bas dans le plan sagittal (l'axe d'implant étant horizontal) sont situés à une distance commune comprise entre environ 2 et 6 mm de l'axe.

Dans un tel jeu, on préfère avoir quatre tailles d'implants phalangiens, chaque implant ayant un corps d'implant avec un diamètre d'extrémité distale de 4,7 ± 2 mm et un diamètre de périphérie de margelle de 12 ± 1, 13,3 ± 1, 14,6 ± 1 et 16 ± 1 mm.

On peut prévoir alors trois ou quatre plots de tailles différentes, à savoir des plots de diamètre de 12 ± 0,5, 13 ± 0,5 et 14 ± 0,5 mm pour un jeu de trois plots.

L'invention a également pour objet un ensemble d'ancillaires permettant la pose de la prothèse selon l'invention.

Cet ensemble d'ancillaire comporte avantageusement les pièces suivantes :
- un ancillaire de repérage présentant un corps muni d'un passage interne cylindrique lui permettant de coulisser et de tourner autour d'une broche implantée axialement dans le métatarsien, et, à partir dudit corps, deux bras coudés dont les extrémités sont agencées dans l'espace pour pouvoir venir sensiblement s'immobiliser dans les gorges sésamoïdiennes, vers leur extrémité distale à la partie inférieure du métatarsien, de façon à maintenir le corps dans une position angulaire convenable par rapport auxdites deux gorges sésamoïdiennes, ledit corps présentant, en outre, un moyen de réception et de positionnement d'un ancillaire de coupe métatarsienne, ce moyen pouvant être avantageusement une tige de préférence non circulaire, s'étendant parallèlement à l'axe de l'implant et donc à la broche, et à distance du métatarsien dans la direction latérale vers le plan sagittal du patient.
- un ancillaire de guidage de coupe métatarsienne présentant un corps avec un passage lui permettant de coulisser sur le moyen de réception de l'ancillaire de repérage, ledit corps présentant des moyens de fixation par rapport à l'os, de préférence des passages transversaux susceptibles d'être traversés par de petites broches de fixation latérales que l'on vient visser dans la corticale latérale du métatarsien, ledit corps présentant, en outre, au moins une et de préférence deux fentes de coupe pour le passage d'une scie oscillante, ladite au moins une fente étant inclinée d'un angle à 70° ± 20°, par rapport à l'axe de la broche lorsque l'ensemble est monté sur le métatarsien, la deuxième fente étant plus inclinée de façon que, lesdites deux fentes permettant ainsi de réaliser d'une part la coupe recevant la face arrière de la tête d'implant et la deuxième coupe qui forme un angle obtus, de préférence de 145° ± 20° avec la coupe précitée ;
- un ancillaire de coupe phalangienne présentant un corps lui permettant d'être fixé également par de petites broches, latéralement sur la corticale de la phalange, au moins une fente et de préférence deux fentes parallèles de coupe phalangienne, et un prolongement destiné à venir s'appliquer, de préférence par emboîtement, sur une extrémité correspondante de l'ancillaire de coupe métatarsien, de façon à définir exactement les positions relatives des deux ensembles de coupe, les fentes de coupe métatarsienne et phalangienne étant, lorsque les deux ancillaires de coupe sont réunis, sensiblement parallèles.

D'autres avantages et caractéristiques de l'invention apparaîtront avec la description suivante faite à titre d'exemple non limitatif et se référant au dessin annexé dans lequel :
la figure 1 représente, schématiquement une vue en perspective d'un implant selon l'invention posé,
la figure 2 représente, de façon schématique, en coupe sagittale de la position de trois implants métatarsiens d'un jeu par rapport à un métatarsien,
la figure 3 représente, en coupe schématique, un implant métatarsien avec son plot,
les figures 4 à 9 représentent des vues respectives de dessus et de côté de trois implants métatarsiens de tailles différentes,
les figures 10 à 12 représentent, en élévation, trois plots de tailles différentes,
les figures 13 à 16 représentent, en élévation, quatre implants phalangiens de tailles différentes,
la figure 17 représente une vue schématique en élévation de l'ancillaire de repérage,
la figure 18 représente une vue de dessous de cet ancillaire,
la figure 19 représente une vue schématique d'une extrémité de métatarsien avec des positions angulaires différentes de l'ancillaire de repérage par rapport aux gorges sésamoïdes,
la figure 20 représente une vue en élévation de l'ancillaire de coupe métatarsienne lors de sa mise en place,
la figure 21 représente une vue de dessous lors de la fixation de cet ancillaire,
la figure 22 représente une vue latérale de l'ancillaire de coupe phalangienne positionné sur l'ancillaire de coupe métatarsien,
la figure 23 représente une vue de dessous de l'assemblage de la figure 22.

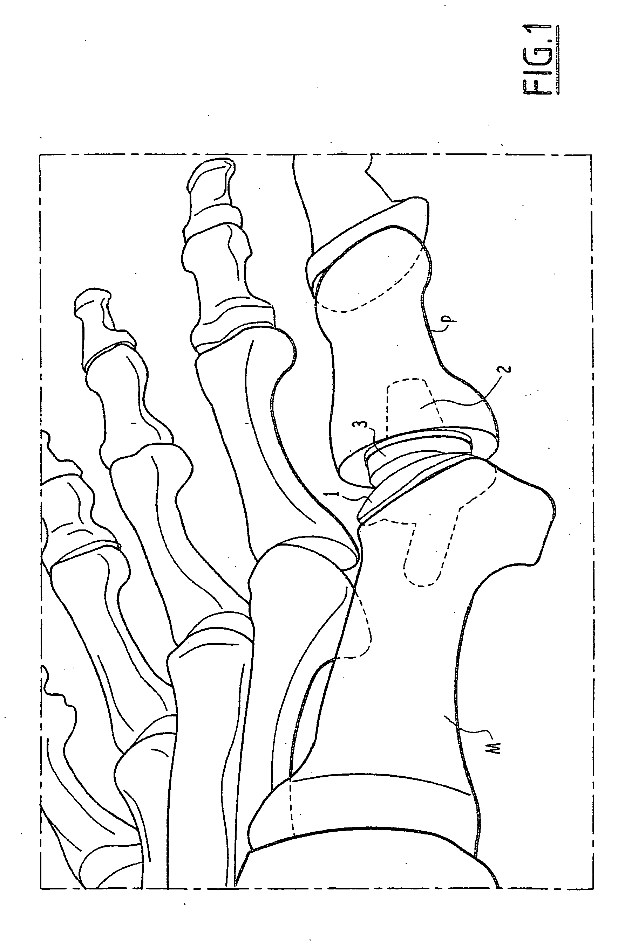

En se référant à la figure 1, on voit un métatarsien M et une phalange P ayant reçu, après mise en place des coupes correspondantes, un implant métatarsien 1, et un implant phalangien 2 avec un plot formant surface d'articulation 3.

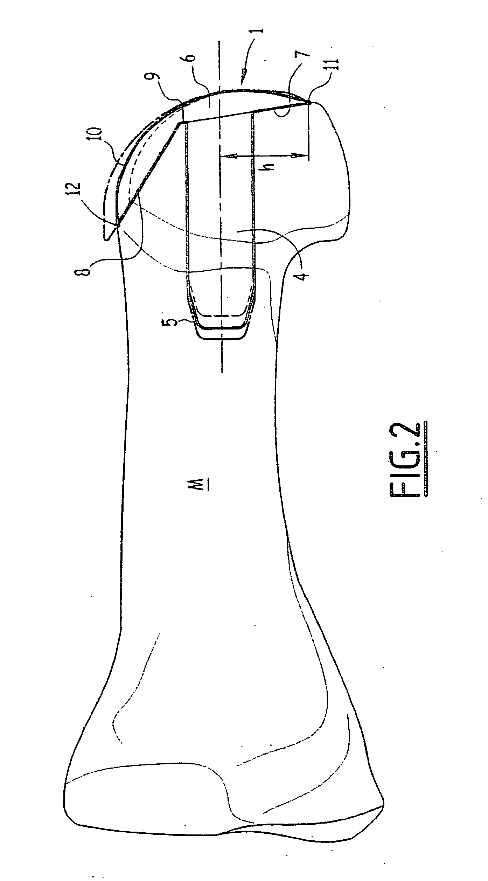

En se référant à la figure 2, on voit en trait plein, une section schématique de l'implant métatarsien
1. Les traits pointillés et mixtes représentent respectivement deux autres tailles d'implants métatarsiens disposés dans la même position sur le métatarsien M, les trois implants métatarsiens formant un exemple d'un jeu d'implants métatarsiens selon l'invention.

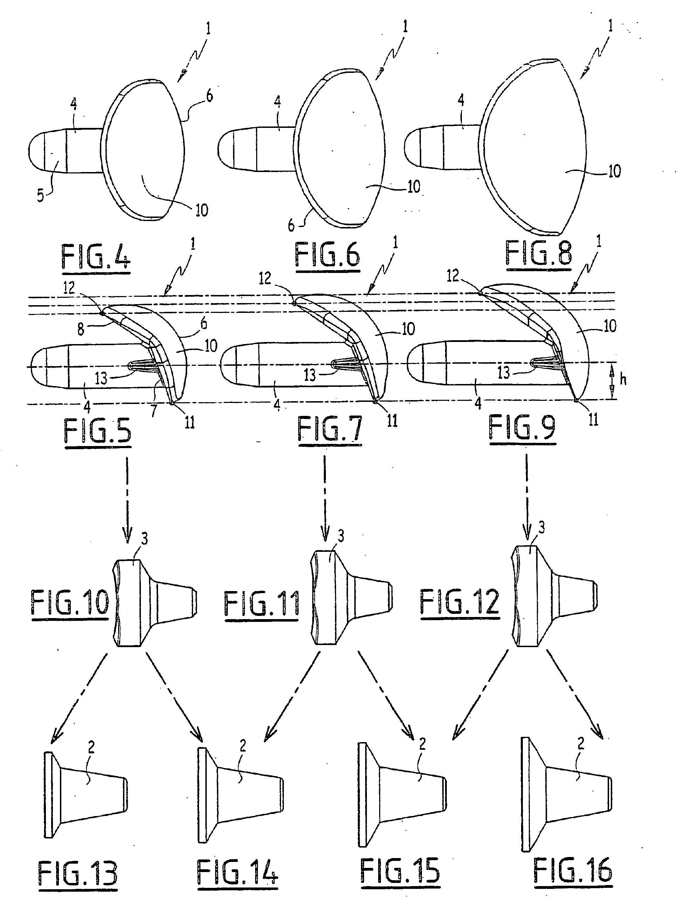

Comme on le voit également en se référant aux figures 4 à 9, chaque implant métatarsien 1 présente une tige cylindrique 4 se terminant par une extrémité chanfreinée puis arrondie 5. Le diamètre de cette tige est destiné à permettre la fixation de l'implant dans un perçage préalablement pratiqué axialement dans le métatarsien.

L'implant métatarsien présente également une tête 6 présentant deux faces formant un angle diédrique. L'une est la face arrière 7 qui forme avec l'axe de la tige 4 un angle de 20° environ, de façon à ce que la face arrière soit inclinée vers le haut et vers l'arrière. A peu près au niveau de l'encombrement supérieure de la tige 4, la face plane 7 se poursuit par une face 8 inclinée d'un angle d'environ 145° par rapport à la face 7. Dans l'exemple représenté la tige 4 vient se raccorder intégralement à la face 7 mais on comprend que l'axe 9 du dièdre pourrait également être plus bas ou plus haut.

Du côté distal, la tête 6 présente une surface articulaire 10 qui, dans le plan sagittal représenté, possède un rayon de courbure unique pour former un arc de cercle. Dans le plan perpendiculaire, c'est à dire dans le plan horizontal, la surface 10 présente également un rayon de courbure unique. La surface articulaire 10 vient se raccorder à la face arrière 7, dans le plan sagittal, en un point inférieur 11 situé à une distance h de l'axe de la tige 4.

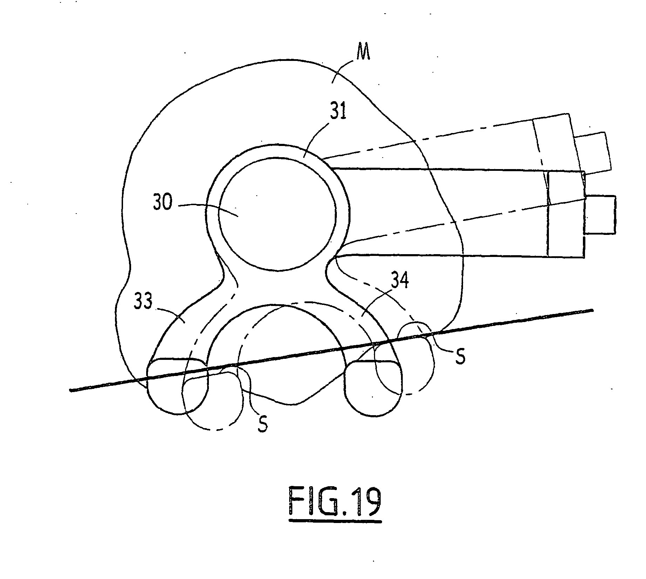

De préférence, dans le cadre de l'invention, cette distance h est choisie entre 2 et 6 mm. Cette distance h est identique ou sensiblement identique pour tous les implants métatarsiens d'un même jeu. La distance h est telle que lorsque l'implant est mis en position comme représenté sur la figure 2, sur les métatarsiens, la position du point 11 soit telle que la surface articulaire, dans sa partie inférieure, ne vienne pas empiéter sur le débouché des gorges sésamoïdes S représentées sur la figure 19.

De plus, on voit que selon les dimensions des faces articulaires 10 des implants métatarsiens du jeu représenté sur les figures 4 à 9, le point haut 12 de l'implant formé par la rencontre des surfaces 8 et 10 peut, grâce au choix de la dimension de l'implant, déborder ou non au-delà de l'encombrement supérieur du métatarsien M et s'adapter au mieux à l'état ligamentaire local.

Afin d'éviter des risques de rotation de l'implant métatarsien dans le métatarsien, la tige de l'implant peut encore comprendre des moyens d'immobilisation en rotation, telles que des ailettes radiales 13 situées au niveau de la face 8.

On voit encore sur la figure 2, que lorsque l'implant est mis en place à l'extrémité du métatarsien, la face arrière 7 vient s'appliquer contre la face de coupe correspondante, de même inclinaison, à l'extrémité du métatarsien, alors que la face inclinée 8 vient s'appliquer contre une autre face, inclinée de façon correspondante, d'une coupe de l'extrémité du métatarsien.

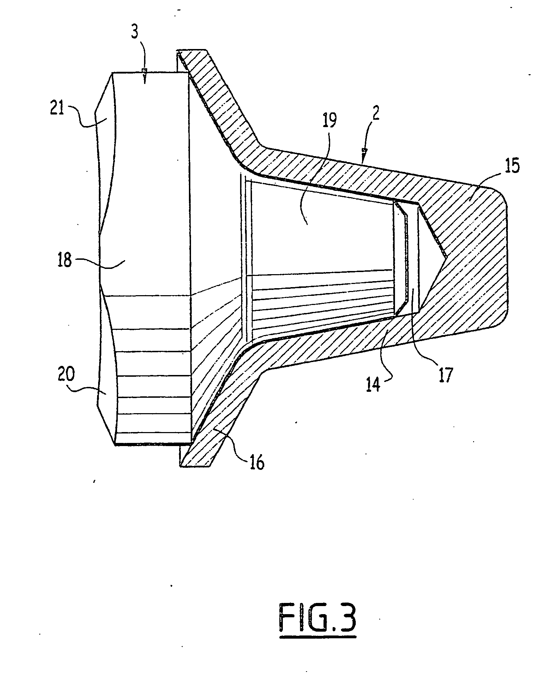

En se référant à la figure 3, on voit l'implant phalangien 2 qui présente un corps tronconique 14 ayant une petite base distale 15 et se poursuivant, à son extrémité proximale, par une margelle conique 16. Dans le corps 14, et dans le prolongement de la face proximale de la margelle 16, est disposé un évidement tronconique 17.

De façon avantageuse, le diamètre de la partie 15 est faible, par exemple de l'ordre de 4,7 mm. A ce niveau, dans le débouché de l'évidement 17, le diamètre interne est avantageusement de 6 cm, l'évidemment tronconique dans le corps 14 permettant de recevoir des plots de 6 mm par exemple.

Le diamètre maximum de la margelle 16 peut également varier en fonction de la taille de l'implant phalangien.

Cet implant phalangien peut recevoir le plot 3 comprenant un plateau 18 et une queue 19. La face distale du plateau 18 est congruente à la face conique interne de la margelle 16, alors que la surface externe de la queue 19 est congruente et de même conicité que l'évidemment 17 dans le corps d'implant 14. On comprend, que de cette façon, le plot 3 est reçu dans l'implant et présente une forme lui conférant une résistance particulière. On peut, par exemple, obtenir avec des plots en polyéthylène, des résistances au cisaillement d'environ 42 daN.

La queue du plot présente toujours la même dimension alors que le diamètre et/ou l'épaisseur du plateau 18 peuvent varier.

La face articulaire proximale du plot, comme on le voit sur la figure 1, est concave et congruente à la surface articulaire de l'implant métatarsien, c'est-à-dire que cette face présente, dans des plans perpendiculaires, des rayons de courbure respectivement constants et identiques à ceux de la surface de l'implant métatarsien.

On voit, enfin, que l'on a pratiqué en périphérie de la face articulaire du plot deux entailles ou chanfrein, à savoir un chanfrein inférieur 20 et un chanfrein supérieur 21 qui s'étendent sur presque toute la moitié de l'arc de cercle périphérique concerné, ces chanfreins permettant un dégagement supplémentaire en flexion, respectivement en extension.

En se référant plus précisément aux figures 4 à 16, on a représenté un jeu complet comprenant trois implants métatarsiens 1, quatre implants phalangiens 2, et trois plots 3.

Le diamètre des tiges 4 est, pour chaque implant métatarsien, de 5 mm environ, par exemple à plus ou moins 2 mm. Les rayons de courbure, comme cela est indiqué sur les dessins, dans le plan sagittal sont, par taille croissante, de 7 ± 0,5, de 8 ± 0,5 et de 9 ± 0,5 mm et, dans le plan frontal, de 14, de 16 et de 18 mm. Les encombrements dans le plan horizontal sont de 16, 18 et 20 mm.

Les quatre implants phalangiens 2 du jeu ont respectivement des diamètres extérieurs de margelle de 12 ± 1, 13,3 ± 1 , 14,6 ± 1 et 16 ± 1 mm. Les logements et surfaces recevant les plots sont identiques.

Le jeu comporte enfin trois plots 3 dont les diamètres maximum de plateau sont respectivement de 12 ± 0,5, 13 ± 0,5 et 14 ± 0,5 mm.

Toutes les combinaisons implant métatarsien/plot/ implant phalangien sont envisageables grâce à l'adaptabilité des composants. On a représenté sur la planche comprenant les figures 4 à 16, par des flèches en traits mixtes, les correspondances médicalement préférées entre les trois constituants, à savoir les implants métatarsiens, les plots et les implants phalangiens.

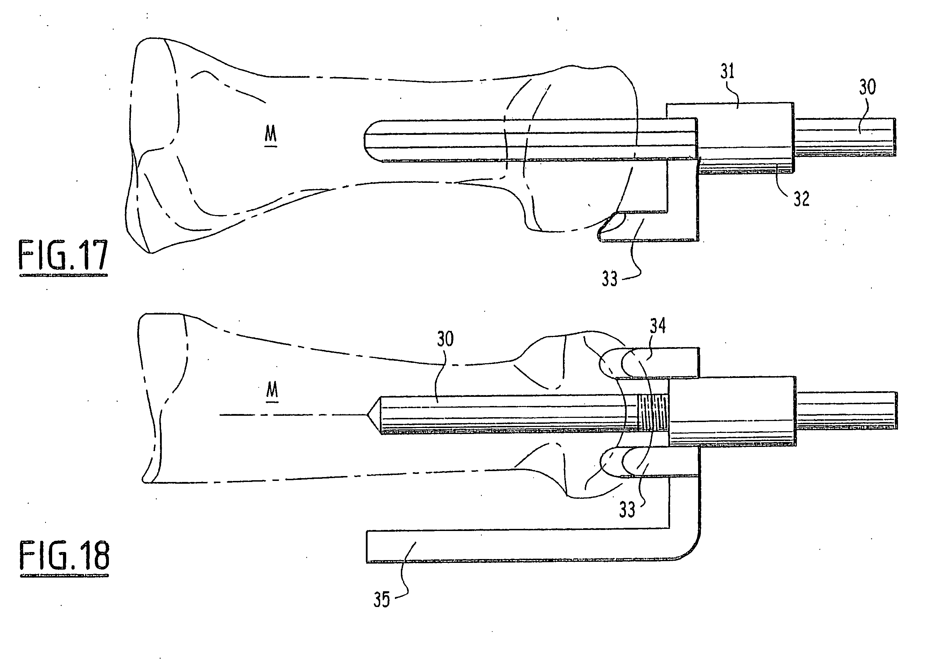

On se réfère, maintenant aux figures 17 à 19.

Pour poser une prothèse selon l'invention, après avoir luxé la phalange, on aborde l'extrémité distale du métatarsien M et on réalise un perçage axial dans le centre de la surface articulaire pathologique à l'aide d'une broche filetée 30 que l'on laisse en place.

On enfile ensuite sur la broche un ancillaire de repérage 31 capable de glisser et de tourner sur la broche et présentant, à cet effet, un corps 32 muni d'un passage correspondant. A son extrémité proximale, le corps 32 présente deux bras 33 et 34 coudés, d'abord latéralement puis vers le bas, puis vers l'avant, dont les extrémités sont émoussées et s'étendent de façon oblique vers le bas. L'écartement entre les deux extrémités des bras 33 et 34 correspond sensiblement à la distance entre les deux gorges sésamoïdes S lorsqu'elles débouchent à la face antérieure articulaire du métatarsien. Du corps 31 s'étend également un autre bras coudé allongé 35, dont la plus grande partie s'étend parallèlement à la broche 30 et à une certaine distance de la surface latérale du métatarsien.

On enfile la pièce 31 sur la broche 30 et on la fait coulisser et tourner convenablement pour l'amener dans la position dans laquelle les deux extrémités des bras 33 et 34 pénètrent dans le début des gorges sésamoïdes S jusqu'à ce que toute rotation soit empêchée, ce qui fixe à la fois la position axiale et la position en rotation de l'ancillaire 31.

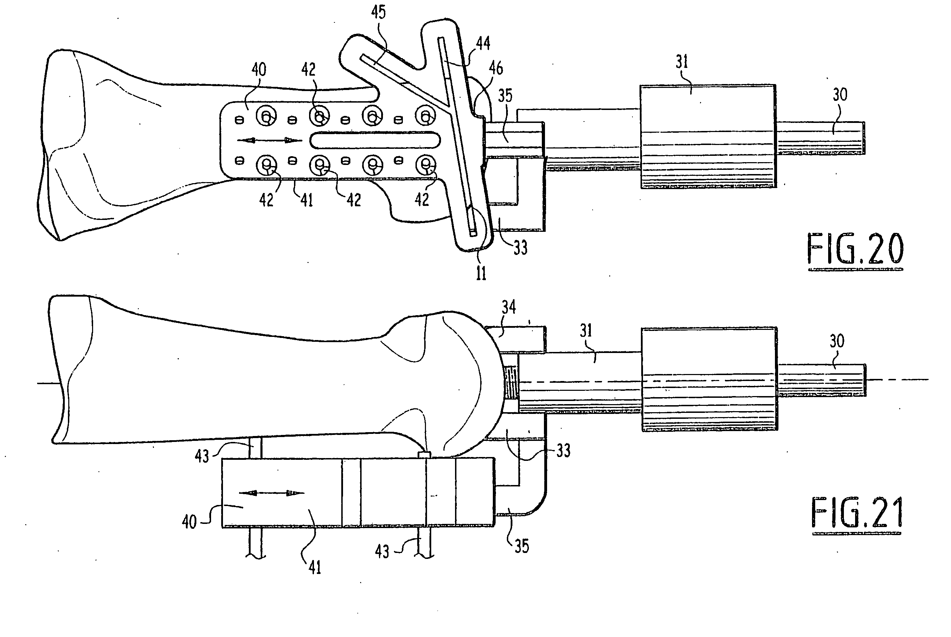

On se réfère maintenant aux figures 20 et 21.

On enfile, alors dans l'autre sens, sur le bras 35, un ancillaire de coupe métatarsienne 40 qui présente un corps rectangulaire 41 muni d'un passage allongé recevant le bras 35 sur lequel l'ancillaire 40 peut coulisser mais sans pivoter.

Le corps 41 présente une pluralité de trous transversaux 42 par lesquels pourront passer des petites broches 43 qui permettront de fixer l'ancillaire 40 sur le métatarsien M. Vers sa partie antérieure le corps 41 présente des élargissements dans lesquels est pratiquée une première fente transversale 44, inclinée de 70° par rapport au corps 40 et au bras 35 et donc à la broche 30. Un peu au-dessous du dernier tiers supérieur de la fente 44, débute une deuxième fente 45 oblique vers le haut et formant avec la fente 44 un angle obtus.

Enfin le corps 41 présente, à son extrémité distale, une surface 46 présentant un petit logement interne.

On fait coulisser l'ancillaire 40 sur le bras 35 jusqu'à ce que la fente 44 vienne, dans son plan transversal, en alignement avec la partie supérieure de l'extrémité du bras 33, comme on le voit sur la figure 20, ce qui détermine le point 11 d'encombrement inférieur de l'implant métatarsien qui va être posé. Dans cette position on visse les broches 43 et l'on fixe rigidement le corps 41 par rapport au métatarsien. Ensuite, on retire l'ancillaire 31, puis la broche 30, dont le diamètre correspond au diamètre de la tige 4 de l'implant métatarsien, qui pourra ultérieurement être impacté dans le trou laissé par une broche.

Enfin, on assure la coupe de l'extrémité du métatarsien à l'aide d'une scie vibrante, d'abord par la fente 44, puis par la fente 45, ce qui crée à l'extrémité antérieure les deux plans de coupe correspondants, et inclinés selon les inclinaisons des fentes.

On peut ensuite introduire une prothèse métatarsienne d'essai similaire à la prothèse métatarsienne définitive, ce qui permet, dans le jeu de prothèses, de choisir la taille la plus adéquate.

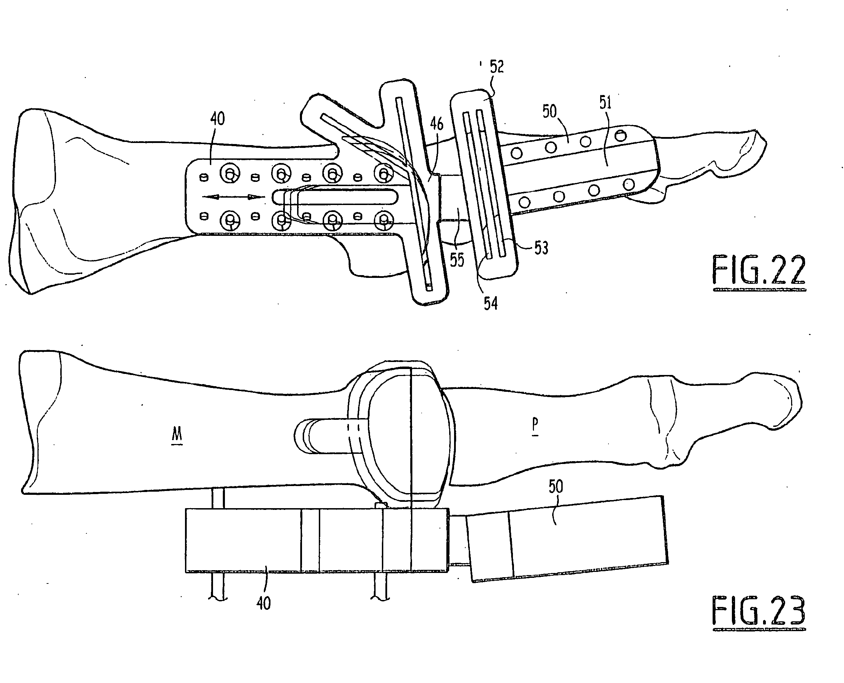

L'implant 1 métatarsien d'essai étant en place, on réduit la luxation et on ramène la phalange P dans la position naturelle avec sa surface articulaire contre la surface articulaire de l'implant métatarsien d'essai, comme on le voit bien notamment sur la figure 23.

Comme représenté sur les figures 22 et 23, on vient alors mettre en place l'ancillaire de coupe phalangienne 50. Cet ancillaire présente un corps 51, également muni d'une pluralité de trous qui permettront le passage de petites broches de fixation sur la phalange. Vers son extrémité proximale, ce corps 51 présente un élargissement 52 dans lequel sont disposées deux fentes parallèles 53 et 54. L'extrémité proximale de cet élargissement se poursuit par une terminaison 55 qui présente un court embout lui permettant de pénétrer dans le petit logement de l'extrémité 46 de l'ancillaire métatarsien 40, ce qui détermine exactement les positions respectives des deux ancillaires 40 et 50. Comme on le voit sur la figure 23, dans cette position le corps 50 est légèrement incliné pour reproduire la légère position naturelle en varus de la première phalange P. On peut alors, en utilisant des broches similaires aux broches 43, fixer le corps 50 sur la face latérale de la phalange P. Dans cette position on voit que les fentes 53 et 54 sont parallèles à la fente 44 de l'implant métatarsien.

Le chirurgien utilise alors l'une des deux fentes 53 et 54 pour pratiquer une coupe droite de l'extrémité de la phalange à l'aide d'une lame oscillante ou vibrante.

Ensuite, on dépose, par retrait des broches correspondantes, les deux implants de coupe 40 et 50.

On effectue ensuite à une nouvelle luxation de la phalange et on procède au choix de la dimension d'implant phalangien.

De façon optionnelle, on peut utiliser pour cela un gabarit formé d'un disque situé, à l'extrémité d'un manche dans le plan du disque, présentant éventuellement en son intérieur un trou central, ce disque permettant de déterminer le diamètre de la coupe phalangienne.

Eventuellement, ce gabarit peut être mis en place à l'aide d'une broche que l'on fait passer à travers son trou central, de façon à être disposé dans la bonne position dans la phalange.

Après retrait du gabarit, on procède ensuite au fraisage de la phalange pour pratiquer le trou qui recevra l'implant phalangien. Pour cela on utilise une fraise ayant une forme extérieure et conique à deux étages correspondant aux deux étages coniques de l'implant. Le cas échéant, cette fraise peut être guidée par une broche laissée préalablement en place et ayant servi lors du positionnement du gabarit.

Après fraisage, on peut mettre en place un implant phalangien d'essai, puis, après retrait de celui-ci, on impacte définitivement l'implant phalangien 2 choisi.

On extrait ensuite l'implant d'essai métatarsien, que l'on remplace par l'implant métatarsien définitif correspondant, que l'on impacte dans le métatarsien M.

Il reste ensuite à choisir la dimension du plot et, notamment, l'épaisseur du plateau du plot pour obtenir le bon écartement entre la phalange et le métatarsien. Une fois le plot mis en place dans l'implant phalangien, on réduit la luxation et on procède à la fermeture des plans anatomiques.

Divers aménagements et variantes aux ancillaires décrits ci-dessus sont en outre envisageables, notamment en ce qui concernent le nombre et la disposition des fentes de coupe.

## Revendications

1. Prothèse totale métatarso-phalangienne comprenant
un implant métatarsien (1) comprenant une tige d'ancrage (4) destinée à être disposée longitudinalement dans le métatarsien (M), et une tête (6) située à l'extrémité de la tige, destiné à être appliquée contre la surface de la coupe de l'extrémité du métatarsien, et présentant une surface articulaire (10),
et un implant phalangien (2) présentant un corps (14) destiné à être introduit axialement dans la phalange et une margelle (16) située à l'extrémité proximale de ladite partie et susceptible de présenter une surface articulaire (18) complémentaire de la surface articulaire (10) présentée par le corps de l'implant métatarsien,
**caractérisé en ce que** la tête (6) de l'implant métatarsien présente, à l'opposé de la surface articulaire (10), une face plane arrière inclinée (7), destinée à venir s'appliquer contre une surface de coupe du métatarsien (M), ladite face arrière (7) formant, avec le plan perpendiculaire à l'axe de la tige d'ancrage (4), un angle de 20° ± 5° pour s'étendre en arrière vers le haut, depuis la base inférieure du métatarsien,
et **en ce que** la surface articulaire (10) de ladite tête et ladite face arrière inclinée (7) se rencontrent en un point inférieur (11), formant le point le plus bas de l'implant métatarsien, la distance entre l'axe de la tige d'ancrage (4) et ledit point le plus bas (11) étant déterminée pour que la tête d'implant (6) ne s'étende pas dans les gorges sésamoides disposées sur la base inférieure du métatarsien.

2. Prothèse selon la revendication 1, **caractérisée en ce que** ladite surface articulaire (18) de l'implant phalangien (2) est portée par un plot (3) distinct susceptible d'être introduit et maintenu dans l'implant phalangien (2).

3. Prothèse selon l'une des revendications précédentes, **caractérisée en ce que** la surface articulaire (10) du corps de l'implant ne présente qu'un seul rayon dans le plan sagittal et un seul rayon dans le plan horizontal, la face articulaire (18) de l'implant phalangien étant congruente et portée par un plot (3) susceptible de tourner autour de l'axe de l'implant phalangien.

4. Prothèse selon l'une des revendications 2 ou 3, **caractérisée en ce que** la surface articulaire (18) du plot (3) présente sur sa périphérie, notamment sur une partie sensible de la périphérie de la moitié inférieure et/ou de la périphérie de la moitié supérieure, une échancrure (20, 21) déterminant un rebord convexe pour le dégagement de l'articulation en position de flexion et/ou d'extension.

5. Prothèse selon l'une des revendications 2 à 4, **caractérisée en ce que** l'implant phalangien (2) présente un corps d'ancrage tronconique creux (14) se poursuivant, à son extrémité proximale, par une tête en forme de margelle tronconique périphérique (16), le plot (3) présentant une forme correspondante, à savoir une partie distale tronconique (19) dont la conicité correspond à celle du corps, se poursuivant par un plateau (18) ayant une surface distale périphérique dont la conicité correspond à celle de la margelle (16) de l'implant, ce qui permet également de donner au plot une forme particulièrement résistante.

6. Prothèse selon l'une des revendications précédentes, **caractérisée en ce que** la face proximale (7) inclinée à 20° ± 5° de la tête (6) de l'implant métatarsien se prolonge, vers le haut, par une face supérieure (8) formant, avec la précédente, un dièdre, de préférence d'un angle obtus de 145° ± 20°, destiné à être appliqué contre un plan de coupe supérieur incliné du même angle par rapport à la coupe recevant la face proximale (7).

7. Jeu d'implants métatarsiens comprenant plusieurs implants métatarsiens (1) de tailles différentes, adaptés pour former une partie d'une prothèse conforme à l'une quelconque des revendications 1 à 6, la distance (h) entre l'axe de la tige d'ancrage (4) et le point le plus bas (11) étant sensiblement constante pour tous les implants du jeu.

8. Jeu d'implants suivant la revendication 7, **caractérisé en ce que** les implants métatarsiens (1) présentent les caractéristiques de la revendication 6, les faces articulaires (10) des têtes (6) de ces implants étant dimensionnées pour rencontrer la face supérieure correspondante (8) en un point supérieur (12) à des distances respectives prédéterminées différentes de l'axe de la tige d'ancrage (4).

9. Jeu d'implants selon l'une des revendications 7 ou 8, **caractérisé en ce que** le diamètre de la tige (4) des implants métatarsiens (1) est sensiblement constant.

10. Jeu de prothèses qui comprend un jeu d'implants métatarsiens (1) conforme à l'une quelconque des revendication 7 à 9, plusieurs implants phalangiens (2) de tailles différentes et plusieurs plots (3), de tailles également différentes, **caractérisé par le fait que** le plot (3) dont la surface articulaire (18) est congruente avec la surface articulaire (18) d'un implant métatarsien donné, peut être reçu dans, au moins, deux implants phalangiens de tailles consécutives pour former une prothèse conforme à l'une quelconque des revendications 1 à 7.

11. Jeu selon la revendication 9, **caractérisé en ce que** l'implant phalangien (2) présente un corps tronconique (14) ayant la même dimension pour tous les implants dans le jeu, ce cône étant suivi de la margelle tronconique (16), seul le diamètre de la périphérie de cette margelle (16) étant variable d'un implant à l'autre dans le jeu de façon.

12. Jeu selon l'une des revendications 10 ou 11, comprenant trois implants métatarsiens (1), **caractérisé en ce que** les rayons de courbure de la surface articulaire (10) sont respectivement de 7 ± 0,5, 8 ± 0,5 et 9 ± 0,5 mm dans le plan sagittal et 14 ± 1, 16 ± 1 et 18 ± 1 mm dans le plan horizontal, et les points les plus bas (11) dans le plan sagittal sont situés à une distance commune (h) comprise entre environ 2 et 6 mm de l'axe.

13. Jeu selon la revendication 12, **caractérisé par** quatre tailles d'implants phalangiens (2), chaque implant ayant un corps d'implant (14) avec un diamètre d'extrémité distale de 4,7 ± 2 mm et un diamètre de périphérie de margelle (16) de 12 ± 1, 13,3 ± 1, 14,6 ± 1 et 16 ± 1 mm.

14. Jeu selon la revendication 13, **caractérisé en ce qu'**il comporte trois plots (3) de tailles différentes, à savoir des plots de diamètre de 12 ± 0,5, 13 ± 0,5 et 14 ± 0,5 mm pour un jeu de trois plots.

15. Ancillaire de repérage (31) pour la pose d'une prothèse selon l'une des revendications 1 à 6 présentant un corps (32) muni d'un passage interne cylindrique lui permettant de coulisser et de tourner autour d'une broche implantée axialement dans le métatarsien, et, à partir dudit corps (32), deux bras coudés (33, 34) dont les extrémités sont agencées dans l'espace pour pouvoir venir sensiblement s'immobiliser dans les gorges sésamoïdiennes, vers leur extrémité distale à la partie inférieure du métatarsien, de façon à maintenir le corps (32) dans une position axiale et angulaire convenable par rapport auxdites deux gorges sésamoïdiennes, ledit corps (32) présentant, en outre, un moyen (35) de réception et de positionnement d'un ancillaire de coupe métatarsienne, s'étendant parallèlement à l'axe de l'implant et donc à la broche, et à distance du métatarsien dans la direction latérale vers le plan sagittal du patient.

16. Ancillaire selon la revendication 15, **caractérisé en ce que** ledit moyen de réception (35) est une tige de section non circulaire.

17. Ancillaire (40) de guidage de coupe métatarsienne pour la pose d'une prothèse selon l'une des revendications 1 à 6 présentant un corps (41) avec un passage lui permettant de coulisser sur le moyen de réception un ancillaire de repérage (31) selon l'une des revendications 15 et 16, ledit corps présentant des moyens (42) de fixation par rapport à l'os, ledit corps (41) présentant, en outre, au moins une fente de coupe pour le passage d'une scie oscillante, ladite fente (44) étant inclinée d'un angle à 70° ± 20°, par rapport à l'axe de la broche lorsque l'ensemble est monté sur le métatarsien.

18. Ancillaire selon la revendication 17, **caractérisé en ce qu'**il comporte une deuxième fente (45) plus inclinée de façon que, lesdites deux fentes (44, 45) permettent ainsi de réaliser d'une part la coupe recevant la face arrière (7) de la tête (6) d'implant et la deuxième coupe qui forme un angle obtus, de préférence de 145° ± 20° avec la coupe précitée.

19. Ancillaire (50) de coupe phalangienne pour la pose d'une prothèse selon l'une des revendications 1 à 6 présentant un corps (51) lui permettant d'être fixé, latéralement sur la corticale de la phalange, au moins une fente (53) de coupe phalangienne, et un prolongement (55) destiné à venir s'appliquer, contre une extrémité correspondante de l'ancillaire de coupe métatarsien (40) selon l'une des revendications 17 et 18, de façon à définir exactement les positions relatives des deux ensembles de coupe, les fentes de coupe métatarsienne et phalangienne étant, lorsque les deux ancillaires de coupe sont réunis, sensiblement parallèles.

20. Ancillaire selon la revendication 19, **caractérisé en ce qu'**il comporte deux fentes de coupe parallèles (53, 54).

## Claims

1. Total metatarsophalangeal prosthesis comprising
a metatarsal implant (1) comprising an anchoring bar (4) intended to be located longitudinally in the metatarsal (M), and a head (6) located at the end of the bar intended to be applied against the cut surface of the end of the metatarsal and having an articular surface (10),
and a phalangeal implant (2) having a body (14) intended to be inserted axially into the phalange, and a collar (16) located at the proximal end of said part and suitably configured to have an articular surface (18) complementary to the articular surface (10) presented by the body of the metatarsal implant, **characterised in that** opposite the articular surface (10) the head (6) of the metatarsal implant has an inclined plane rear face (7) intended to come to rest against a cut surface of the metatarsal (M), said rear face (7) forming an angle of 20° ± 5° with the plane perpendicular to the axis of the anchoring bar (4) to extend to the rear and upwards from the lower base of the metatarsal,
and **in that** the articular surface (10) of said head and said inclined rear face (7) meet at a lower point (11) to form the lowest point of the metatarsal implant, the distance between the axis of the anchoring bar (4) and said lowest point (11) being determined such that the implant head (6) does not extend into the sesamoid grooves located on the lower base of the metatarsal.

2. Prosthesis according to Claim 1, **characterised in that** said articular surface (18) of the phalangeal implant (2) is supported by a separate stud (3) capable of being inserted and held in the phalangeal implant (2).

3. Prosthesis according to one of the preceding claims, **characterised in that** the articular surface (10) of the body of the implant only has a single radius in the sagittal plane and a single radius in the horizontal plane, the articular surface (18) of the phalangeal implant being congruent and supported by a stud (3) capable of rotating around the axis of the phalangeal implant.

4. Prosthesis according to one of Claims 2 or 3, **characterised in that** on its periphery, in particular on a sensitive section of the periphery of the lower half and/or the periphery of the upper half, the articular surface (18) of the stud (3) has a bevel (20, 21) defining a convex edge for clearance for articulation in the flexion and/or extension position.

5. Prosthesis according to one of Claims 2 to 4, **characterised in that** the phalangeal implant (2) has a hollow tapered anchoring body (14) extended at its proximal end by a head in the shape of a tapered peripheral collar (16), the stud (3) having a corresponding shape, i.e. a tapered distal section (19), the conicity of which corresponds to that of the body, extended by a plate (18) having a distal peripheral surface, the conicity of which corresponds to that of the collar (16) of the implant, and this also allows the stud to be configured particularly solidly.

6. Prosthesis according to one of the preceding claims, **characterised in that** the proximal face (7) inclined at 20° ± 5° of the head (6) of the metatarsal implant is extended upwards by way of an upper face (8) and with this forms a dihedron preferably with an obtuse angle of 145° ± 20° intended to be applied against an upper cutting plane inclined at the same angle relative to the cut receiving the proximal face (7).

7. Set of metatarsal implants comprising several metatarsal implants (1) of different sizes adapted to form a section of a prosthesis in accordance with any one of Claims I to 6, wherein the distance (h) between the axis of the anchoring bar (4) and the lowest point (11) is essentially constant for all the implants of the set.

8. Set of implants according to Claim 7, **characterised in that** the metatarsal implants (1) have the features of Claim 6, wherein the articular faces (10) of the heads (6) of these implants are dimensioned to meet the corresponding upper face (8) at an upper point (12) at respectively different predetermined distances from the axis of the anchoring bar (4).

9. Set of implants according to one of Claims 7 or 8, **characterised in that** the diameter of the bar (4) of the metatarsal implants (1) is substantially constant.

10. Set of prostheses comprising a set of metatarsal implants (1) in accordance with any one of Claims 7 to 9, several phalangeal implants (2) of different sizes and several studs (3) also of different sizes, **characterised in that** the stud (3), of which the articular surface (18) is congruent to the articular surface (18) of a given metatarsal implant, can be received at least in two phalangeal implants of consecutive sizes to form a prosthesis in accordance with any one of Claims 1 to 7.

11. Set according to Claim 9, **characterised in that** the phalangeal implant (2) has a tapered body (14) having the same dimension for all the implants in the set, the tapered collar (16) adjoining this cone, wherein only the diameter of the periphery of this collar (16) is variable from one implant to another in the respective set.

12. Set according to one of Claims 10 or 11, comprising three metatarsal implants (1), **characterised in that** the radii of curvature of the articular surface (10) are respectively 7 ± 0.5, 8 ± 0.5 and 9 ± 0.5 mm in the sagittal plane and 14 ± 1, 16 ± 1 and 18 ± 1 mm in the horizontal plane, and the lowest points (11) in the sagittal plane are located at a common distance (h) in the range of between about 2 and 6 mm from the axis.

13. Set according to Claim 12, **characterised by** four sizes of phalangeal implants (2), wherein each implant has an implant body (14) with a diameter of the distal end of 4.7 ± 2 mm and a diameter of the periphery of the collar (16) of 12 ± 1, 13.3 ± 1, 14.6 ± 1 and 16 ± 1 mm.

14. Set according to Claim 13, **characterised in that** it comprises three studs (3) of different sizes, i.e. studs with a diameter of 12 ± 0.5, 13 ± 0.5 and 14 ± 0.5 mm for a set of three studs.

15. Positioning aid (31) for fitting a prosthesis according to one of Claims 1 to 6, having a body (32) provided with a cylindrical inside passage to allow it to slide and rotate around a pin implanted axially into the metatarsal, and from said body (32) two arms (33, 34), the ends of which are spatially arranged to enable them to lock into the sesamoid grooves, are elbowed towards their distal end at the lower section of the metatarsal in order to hold the body (32) in an appropriate axial and angular position in relation to said two sesamoid grooves, said body (32) additionally having a means (35) for receiving and positioning a metatarsal cutting aid extending parallel to the axis of the implant, and therefore to the pin, and at a distance from the metatarsal in the lateral direction towards the sagittal plane of the patient.

16. Aid according to Claim 15, **characterised in that** said receiving means (35) is a bar with a non-circular cross-section.

17. Aid (40) for guiding a metatarsal cut for fitting a prosthesis according to one of Claims 1 to 6, having a body (41) with a passage to allow it to slide onto the receiving means, a positioning aid (31) according to one of Claims 15 and 16, said body having means (42) for fastening in relation to the bone, said body (41) additionally having at least one cutting slot for passage of an oscillating saw, said slot (44) being inclined at an angle of 70° ± 20° in relation to the axis of the pin when the assembly in mounted on the metatarsal.

18. Aid according to Claim 17, **characterised in that** it comprises a second slot (45) with a greater inclination such that said two slots (44, 45) thus allow the cut to be performed to receive the rear face (7) of the head (6) of the implant as well as the second cut that forms an obtuse angle, preferably 145° ± 20, with the previous cut.

19. Phalangeal cutting aid (50) for fitting a prosthesis according to one of Claims 1 to 6, having a body (51) to allow it to be secured laterally on the cortical area of the phalange, at least one phalangeal cutting slot (53), and an extension (55) intended to be applied against a corresponding end of the metatarsal cutting aid (40) according to one of Claims 17 and 18, in order to precisely define the relative positions of the two cutting assemblies, the metatarsal and phalangeal cutting slots being substantially parallel when the two cutting aids are joined.

20. Aid according to Claim 19, **characterised in that** it comprises two parallel cutting slots (53, 54).

## Patentansprüche

1. Metatarsalphalangeale Vollprothese, mit
einem Metatarsalimplantat (1) mit einer Verankerungsstange (4) für eine Längsanordnung im Mittelfußknochen (M) und einem am Ende der Stange gelegenen Kopf (6), der zur Anbringung an der Oberfläche des Schnittes am Ende des Mittelfußknochens bestimmt ist und eine Gelenkfläche (10) darbietet,
und mit einem phalangealen Implantat (2), das einen Körper (14) zum axialen Einführen in das Zehenglied und eine Einfassung (16) darbietet, die am proximalen Ende des genannten Teils gelegen und geeignet ist, eine Gelenkfläche (18) komplementär zu der vom Körper des metatarsalen Implantats dargebotenen Gelenkfläche (10) darzubieten,
**dadurch gekennzeichnet, daß** der Kopf (6) des Metatarsalimplantats, in Gegenüberlage zur Gelenkoberfläche (10), eine plane hintere Schrägfläche (7) zur Anlage an einer Schnittoberfläche des Mittelfußknochens (M) darbietet, wobei die hintere Fläche (7) mit der zur Achse der Verankerungsstange (4) senkrechten Ebene einen Winkel von 20° ± 5° für eine rückwärtige Erstreckung nach oben von der unteren Basis des Mittelfußknochens aus bildet,
und daß sich die Gelenkoberfläche (10) des Kopfes und die hintere Schrägfläche (7) an einem unteren Punkt (11) treffen, der den tiefsten Punkt des Metatarsalimplantats bildet, wobei der Abstand zwischen der Achse der Verankerungsstange (4) und dem tiefsten Punkt (11) so bestimmt ist, daß sich der Implantatkopf (6) nicht in die auf der unteren Basis des Mittelfußknochens angeordneten Sesamhälse erstreckt.

2. Prothese nach Anspruch 1, **dadurch gekennzeichnet, daß** die Gelenkoberfläche (18) des phalangealen Implantats (2) von einem gesonderten Klotz (3) abgestützt ist, der in das phalangeale Implantat (2) einsetzbar und festsetzbar ist.

3. Prothese nach einem der vorhergehenden Ansprüche, **dadurch gekennzeichnet, daß** die Gelenkoberfläche (10) des Implantatkörpers nur einen einzigen Radius in der Sagittalebene und einen einzigen Radius in der Horizontalebene aufweist und daß die Gelenkfläche (18) des phalangealen Implantats kongruent und von einem um die Achse des phalangealen Implantats drehbaren Klotz (3) abgestützt ist.

4. Prothese nach einem der Ansprüche 2 oder 3, **dadurch gekennzeichnet, daß** die Gelenkoberfläche (18) des Klotzes (3) auf ihrem Umfang, insbesondere auf einem wesentlichen Teil des Umfangs der unteren Hälfte und/oder des Umfangs der oberen Hälfte, einen Ausschnitt (20,21) aufweist, der einen konvexen Rand für das Freimachen des Gelenks in Flexions- und/oder Extensionsposition definiert.

5. Prothese nach einem der Ansprüche 2 bis 4, **dadurch gekennzeichnet, daß** das phalangeale Implantat (2) einen kegelstumpfförmigen hohlen Verankerungskörper (14) aufweist, der sich an seinem proximalen Ende in einen Kopf in Form einer kegelstumpfförmigen Umfangseinfassung (16) fortsetzt, und der Klotz (3) eine entsprechende Form aufweist, nämlich einen distalen kegelstumpfförmigen Teil (19), dessen Konizität derjenigen des Körpers entspricht und der sich in einer Scheibe (18) mit einer distalen Umfangsfläche fortsetzt, deren Konizität derjenigen der Einfassung (16) des Implantats entspricht, was es gleichermaßen ermöglicht, dem Klotz eine besonders haltbare Form zu geben.

6. Prothese nach einem der vorhergehenden Ansprüche, **dadurch gekennzeichnet, daß** sich die mit 20° ± 5° schrägverlaufende proximale Fläche (7) des Kopfes (6) des Metatarsalimplantats nach oben in einer oberen Fläche (8) fortsetzt, die mit der vorhergehenden einen Flächenwinkel, vorzugsweise mit einem stumpfen Winkel von 145° ± 20°, für eine Anlage an einer oberen Schnittebene bildet, die im gleichen Winkel in bezug auf den die proximale Fläche (7) aufnehmenden Schnitt schräg verläuft.

7. Metatarsalimplantatsatz von mehreren Metatarsalimplantaten (1) von unterschiedlicher Größe, geeignet zur Bildung eines Teils einer Prothese nach einem der Ansprüche 1 bis 6, wobei der Abstand (h) zwischen der Achse der Verankerungsstange (4) und dem tiefsten Punkt (11) für sämtliche Implantate des Satzes im wesentlichen konstant ist.

8. Implantatsatz nach Anspruch 7, **dadurch gekennzeichnet, daß** die Metatarsalimplantate (1) die Merkmale des Anspruchs 6 aufweisen, wobei die Gelenkflächen (10) der Köpfe dieser Implantate so bemessen sind, daß sie auf die entsprechende obere Fläche (8) an einem oberen Punkt (12) mit einem jeweils vorherbestimmten unterschiedlichen Abstand von der Achse der Verankerungsstange (4) treffen.

9. Implantatsatz nach einem der Ansprüche 7 oder 8, **dadurch gekennzeichnet, daß** der Durchmesser der Stange (4) der Metatarsalimplantate (1) im wesentlichen konstant ist.

10. Prothesensatz, der einen Metatarsalimplantatsatz (1) nach einem der Ansprüche 7 bis 9, mehrere phalangeale Implantate (2) von unterschiedlicher Größe und mehrere Klötze (3) von ihrerseits unterschiedlicher Größe umfaßt, **gekennzeichnet durch** die Tatsache, daß der Klotz (3), dessen Gelenkoberfläche (18) mit der Gelenkoberfläche (10) eines gegebenen Metatarsalimplantats kongruent ist, in, zumindest, zwei Phalangealimplantaten mit aufeinanderfolgender Größe zur Bildung einer Prothese nach einem der Ansprüche 1 bis 7 aufnehmbar ist.

11. Satz nach Anspruch 9, **dadurch gekennzeichnet, daß** das Phalangealimplantat (2) einen kegelstumpfförmigen Körper (14) mit der gleichen Abmessung für sämtliche Implantate des Satzes aufweist, wobei die kegelstumpfförmige Einfassung (16) auf diesen Kegel folgt und nur der Durchmesser des Umfangs dieser Einfassung (16) von einem Implantat zum anderen in dem Bausatz veränderbar ist.

12. Satz nach einem der Ansprüche 10 oder 11, mit drei Metatarsalimplantaten (1), **dadurch gekennzeichnet, daß** die Krümmungsradien der Gelenkoberfläche (10) jeweils 7 ± 0,5, 8 ± 0,5 und 9 ± 0,5 mm in der Sagittalebene und 14 ± 1, 16 ± 1 und 18 ± 1 mm in der Horizontalebene betragen und die tiefsten Punkte (11) in der Sagittalebene mit einem gemeinsamen Abstand (h) zwischen etwa 2 und 6 mm von der Achse gelegen sind.

13. Satz nach Anspruch 12, **gekennzeichnet durch** vier Größen von Phalangealimplantaten (2), wobei jedes Implantat einen Implantatkörper (14) mit einem Durchmesser am distalen Ende von 4,7 ± 2 mm und einem Umfangsdurchmesser der Einfassung (16) von 12 ± 1, 13,3 ± 1, 14.6 ± 1 und 16 ± 1 mm besitzt.

14. Satz nach Anspruch 13, **dadurch gekennzeichnet, daß** er drei Klötze (3) von unterschiedlicher Größe umfaßt, nämlich Klötze mit einem Durchmesser von 12 ± 0,5, 13 ± 0,5 und 14 ± 0,5 mm für einen Satz von drei Klötzen.

15. Einpassungshilfsgerät (31) zum Einsetzen einer Prothese nach einem der Ansprüche 1 bis 6, mit einem Körper (32), der mit einem zylindrischen Innenkanal versehen ist, der es ihm ermöglicht, um eine in axialer Richtung in den Mittelfußknochen eingesetzte Spindel zu gleiten und umzulaufen, und, ausgehend vom Körper (32), zwei Winkelhebeln (33,34), deren Enden so im Raum angeordnet sind, daß sie sich in den Sesamhälsen zu ihrem distalen Ende hin am unteren Teil des Mittelfußknochens festlegen können, um den Körper (32) in einer passenden axialen und Winkelposition in bezug auf die beiden Sesamhälse zu halten, wobei der Körper (32) außerdem ein Mittel (35) zur Aufnahme und Positionierung eines Hilfsgerätes für einen Metatarsalschnitt aufweist, das sich parallel zur Achse des Implantats und damit der Spindel und mit einem Abstand vom Mittelfußknochen in seitlicher Richtung zur Sagittalebene des Patienten erstreckt.

16. Hilfsgerät nach Anspruch 15, **dadurch gekennzeichnet, daß** das Aufnahmemittel (35) eine Stange mit nicht-kreisförmigem Querschnitt ist.

17. Hilfsgerät (40) zur metatarsalen Schnittführung für die Anbringung einer Prothese nach einem der Ansprüche 1 bis 6, das einen Körper (41) mit einem Kanal aufweist, der es ihm ermöglicht, auf dem Aufnahmemittel eines Einpassungshilfsgeräts (31) nach einem der Ansprüche 15 und 16 zu gleiten, wobei der Körper (41) Fixiermittel (42) in bezug auf den Knochen aufweist und der Körper (41) außerdem zumindest einen Schneidschlitz für den Durchgang einer Pendelsäge aufweist, wobei der Schlitz (44) in einem Winkel von 70° ± 20° in bezug auf die Achse der Spindel geneigt ist, wenn die Einheit auf dem Mittelfußknochen montiert ist.

18. Hilfsgerät nach Anspruch 17, **dadurch gekennzeichnet, daß** es einen schrägeren zweiten Schlitz (45) aufweist, derart, daß es die beiden Schlitze (44,45) somit ermöglichen, einerseits den die hintere Fläche (7) des Implantatkopfes (6) erhaltenden Schnitt und den zweiten Schnitt auszuführen, der einen stumpfen Winkel vorzugsweise von 145° ± 20° mit dem vorgenannten Schnitt bildet.

19. Hilfsgerät (50) für einen Phalangealschnitt zum Einsetzen einer Prothese nach einem der Ansprüche 1 bis 6, mit einem Körper (51), der seitlich auf der Kortikalen des Zehengliedes festlegbar ist, zumindest einem Schlitz (53) für den Phalangealschnitt und einer Verlängerung (55) für eine Anlage an einem entsprechenden Ende des Hilfsgerätes (40) für den Metatarsalschnitt nach einem der Ansprüche 17 und 18, derart, daß die relativen Positionen der beiden Schneideinheiten exakt definiert werden, wobei die Schlitze für den Metatarsal- und den Phalangealschnitt, bei Vereinigung der beiden Schneidhilfsgeräte, im wesentlichen parallel verlaufen.

20. Hilfsgerät nach Anspruch 19, **dadurch gekennzeichnet, daß** es zwei parallele Schneidschlitze (53,54) umfaßt.
